# EUROPEAN PATENT SPECIFICATION

(11) **EP 1 536 839 B1**
(45) Date of publication and mention of the grant of the patent: **15.09.2010**
(21) Application number: 02805705.7
(22) Date of filing: 08.11.2002
(51) Int. Cl.: A61P 31/16

(54) **COMBINATION THERAPY COMPRISING OMEGA-INTERFERON FOR TREATING INFECTION WITH HEPATITIS C VIRUS OR YELLOW FEVER VIRUS**
KOMBINATIONSTHERAPIE MIT OMEGA-INTERFERON ZUR BEHANDLUNG VON HEPATITIS C VIRUS ODER GELBFIEBER VIRUS INFEKTIONEN
THÉRAPIE COMBINÉE COMPRENANT L'INTERFÉRON OMÉGA POUR LA TRAITEMENT DES INFECTIONS DE L'HÉPATITE C VIRUS OU DU VIRUS DE LA FIÈVRE JAUNE

(30) Priority: 09.11.2001 US 337948 P
(43) Date of publication of application: 08.06.2005
(73) Proprietor: Intarcia Therapeutics, Inc, Emeryville CA 94608 (US)
(72) Inventor: MORAN, Mark, S., Orinda, CA 94563 (US); LANGECKER, Peter, Monte Sereno, CA 95030 (US); BLANCHETT, Dennis, G., Danville, CA 94526 (US)
(74) Representative: Walton, Seán Malcolm
(86) International application number: PCT/US2002/036025
(87) International publication number: WO 2003/063573

(56) References cited:
- WO-A-00/40273
- WO-A-02/36072
- WO-A2-00/40273
- "Open-label phase 1B study of hepatitis C viral dynamics with omega interferon treatment - John G McHutchison, Paul J Pockros, Scripps Clinic, La Jolla, CA; Peter Langecker, Dennis Blanchett, William - Lang, Mark Moran, BioMedicines, Inc, Emeryville, CA" HEPATOLOGY, WILLIAMS AND WILKINS, BALTIMORE, MD, US, vol. 34, no. 4, 1 October 2001 (2001-10-01), page A333, XP004716177 ISSN: 0270-9139
- "Open-label phase II study of omega interferon in previously untreated HCV infected patients - Mathias Plauth, Stadisches Klinikum, Dessau Germany; Helga Meisel, Institut fur Medizinische Virologie, Berlin Germany; Peter Langecker, Mark Moran, William Lang, Dennis Blachett, BioMedicines, Emeryville" HEPATOLOGY, WILLIAMS AND WILKINS, BALTIMORE, MD, US, vol. 34, no. 4, 1 October 2001 (2001-10-01), page A331, XP004716169 ISSN: 0270-9139
- PLAUTH MATHIAS MATHIAS ET AL: "Open-label study of omega interferon in previously untreated HCV-infected patients." JOURNAL OF HEPATOLOGY, vol. 36, no. Supplement 1, April 2002 (2002-04), page 125, XP002511882 & BIENNIAL MEETING OF THE INTERNATIONAL ASSOCIATION FOR THE STUDY OF THE LIVER; MADRID, SPAIN; APRIL 15-16, 2002 ISSN: 0168-8278
- "AGA abstracts M1143-M1741" GASTROENTEROLOGY, ELSEVIER, PHILADELPHIA, PA, vol. 122, 1 April 2002 (2002-04-01), pages A278-A347, XP022329236 ISSN: 0016-5085
- HORTON H.M.: 'Antitumor effects of interferon-omega: in vivo therapy of human tumor xenograft in nude mice' CANCER RESEARCH vol. 59, August 1999, pages 4064 - 4068, XP002985518
- DATABASE MEDLINE [Online] US NATIONAL LIBRARY OF MEDICINE (NLM), BETHESDA, MD, US; 1989, STEUHL K P ET AL: "[The significance of bacterial and host factors in corneal infections caused by Pseudomonas aeruginosa]" Database accession no. NLM2507425 & FORTSCHRITTE DER OPHTHALMOLOGIE : ZEITSCHRIFT DER DEUTSCHEN OPHTHALMOLOGISCHEN GESELLSCHAFT 1989, vol. 86, no. 4, 1989, pages 283-286, ISSN: 0723-8045

## Description

### FIELD OF THE INVENTION

The field of the present Invention is the treatment of viral diseases using omega interferon.

### BACKGROUND OF THE INVENTION

The interferons are a group of endogenous peptides produced in response to a number of Infectious or immunological disorders. Endogenous interferons have antiviral, infectious immunomodulatory, or antiproliferative activities. The alpha and beta interferons are known as type I interferons and appear to bind to a common receptor, the so-called α-β receptor. Exogenous interferons, such as recombinant alpha (of various subtypes) or recombinant consensus interferon, have been demonstrated to be useful in the treatment of, for example, viral hepatitis C and certain cancers. A small percentage of patients who are treated with alpha or consensus interferon for periods of several months may no longer manifest positive blood tests for hepatitis C viral ribonucleic acid. Certain cancers may stabilize or shrink In size with interferon treatment.

Such treatment may involve only monotherapy with the interferon, or the interferon may be combined with an adjunctive agent(s). Exogenous recombinant beta interferon (of various subtypes) has been shown to be useful as monotherapy In the treatment of multiple sclerosis. Exogenous recombinant gamma interferon has been shown to be useful as monotherapy in the treatment of chronic granulomatous disease and more recently has been suggested to be useful in the treatment of certain pulmonary disorders. Certain interferons have been chemically modified by the addition of polyethylene polymers and may have enhanced antiviral activity or patient acceptance as a result.

Adjunctive agents administered in conjunction with the interferon may enhance the effectiveness of the treatment using interferons. For example, ribavirin is a non-peptide small molecule which, among other activities, is known to inhibit inosine monophosphate dehydrogenase and has antiviral and immunomodulatory activities. The addition of ribavirin (or other inhibitors of inosine monophosphate dehydrogenase) to an alpha interferon, for example, may increase the long-term response rate in certain patient subgroups with hepatitis C. Other adjuncts to alpha interferon may also be useful in certain clinical settings, interleukin-2, interleukin-2 analogs or derivatives, histamine, histamine analogs or derivatives; monoclonal antibodies; polyclonal antibodies; or any combination thereof.

These currently available antiviral or immunomodulatory therapeutics are, however, not without limitations. For example, the long-term success rate in the treatment of hepatitis C is estimated to be the following: alpha interferon alone (≈ 10-15%); consensus interferon alone (≈ 10-15%); pegylated alpha interferon alone (≈ 20-25%); alpha interferon combined with ribavirin (≈ 30-40%); and alpha interferon plus a histamine-related compound (≈ 30-40%). There is evidence that treatment with the combination of alpha interferon and ribavirin or histamine analogs may induce responses in patients who appeared not to be fully responsive to alpha alone. Consensus interferon at some dose levels has been reported to induce responses in patients who failed to achieve sustained results on lower doses of alpha interferon.

In a large percentage of patients, however, there is no significant response to administration of either alpha or consensus interferon, whether or not combined with another agent (primary viral resistance). In addition, a significant fraction of patients whose disease does respond initially do not have a sustained response after drug therapy ceases (secondary resistance). Among those patients who fail to respond to alpha interferon, the majority also fail to respond to subsequent treatment with consensus interferon. The reasons for primary or secondary resistance are not completely understood but may involve significant variation in blood levels of the interferon, the development of antibodies directed against the interferon, intracellular changes which limit interferon-induced responses, or genetic features of (or other changes in) the virus or the patient or both.

Furthermore, not all patients can tolerate therapy with an interferon, whether alone or in combination with an adjunctive agent, because of adverse side effects. There are clear limitations in the dosing of the alpha, beta, consensus, gamma, leukocyte, and tau interferons, Because the effectiveness of an interferon is dependent upon, for example, the dose administered, any limitation in dosing because of adverse side effects has a further negative clinical consequence: medical utility of the interferon is diminished by the inability to administer higher and more *effective* doses because of the dose-limiting adverse side effects.

Side effects of, for example, alpha interferon include the following (as listed in the current FDA labeling for alpha-2c): headache, fever, fatigue, myalgia, leukopenia, neutropenia, thrombocytopenia, arthralgia, rigors, irritability, nausea, vomiting. Some of the side effects caused by interferons, even at low doses, can be severe, life-threatening, or even fatal. These include, among others, serious infections, seizures, and depression. Suicidal ideation or actual suicide are also associated with prolonged administration of currently marketed interferons.

The occurrence of such side effects can lead frequently to a reduction in interferon dosing or the need to cease treatment altogether. In either circumstance, medical utility is diminished or lost altogether. For example, in one recent study comparing pegylated interferon alpha-2a to unpegylated interferon alpha-2a in 531 patients, dose reduction or discontinuation of therapy was necessary in more than 25% of patients in each treatment group (see PEGINTERFERON ALFA-2a IN PATIENTS WITH CHRONIC HEPATITIS C. Zeuzem S, Feinman SV, Rasenack J et al. New Engl J Med 2000;343:1666-72). It is worth noting, however, that with better pharmacokinetics, the viral response rate at the end of treatment for pegylated alfa was approximately 68% while for unpegylated alfa it was approximately 27% in this particular comparative trial in hepatitis patients. The response rate as judged both by viral response and a reduction in liver enzymes at the end of treatment was even lower, however, 42% and 25%, respectively.

Thus, while present interferon administration offers a useful mode of treatment of certain diseases, significant problems remain regarding tolerability and the overall success of treatment. We have now discovered omega interferon offers a solution to these problems.

### SUMMARY OF THE INVENTION

One aspect of this invention is omega interferon for use in a method of treating an infectious disease In a warm-blooded animal according to the claims. The method may comprise administering to the animal omega Interferon (IFN) at a dosage and activity for the disease state treated sufficient to induce a therapeutic response in the animal, which dosage and activity for the disease state treated is higher than would be well-tolerated based on data for non-omega IFNs.

The omega interferon is administered to such animal in combination with a therapeutically effective amount of at least one adjunctive therapeutic agent as defined in the claims for as long a period of time as the animal tolerates omega interferon, monitoring the levels of a disease marker in the animal during the administration, and continuing the administration of omega Interferon for so long as the levels of the disease marker continue to be favorably changed.

Described herein is an article of manufacture useful for treating an immunologic, proliferative, or infectious disease in a warm-blooded animal subject, which article comprises 1) omega interferon in a form suitable for administering a therapeutically effective amount of the omega IFN to the subject in order to Induce the desired therapeutic response and 2) instructions for administering the omega IFN for the disease state treated at a dosage and activity of omega IFN that is higher than would be well-tolerated based on data for non-omega IFNs.

Also described herein is a process for preparing an omega interferon -based article of manufacture useful for treating an immunologic, proliferative, or infectious disease in a warm-blooded animal subject, which process comprises providing omega IFN as a composition suitable for administering to the subject at a therapeutically effective dosage, and combining the omega IFN so provided with instructions for administering the omega IFN for such disease.

Another aspect of this invention is the use omega interferon (IFN) in the manufacture of a medicament for treating an infectious disease. In a warm-blooded animal as defined in the claims.The medicament is for administration to the animal at a dosage and activity for the disease treated sufficient to induce a therapeutic response in the animal, which dosage and activity for the disease state treated is higher than would be well-tolerated based on data for non-omega IFNs.

### Description of the Figures

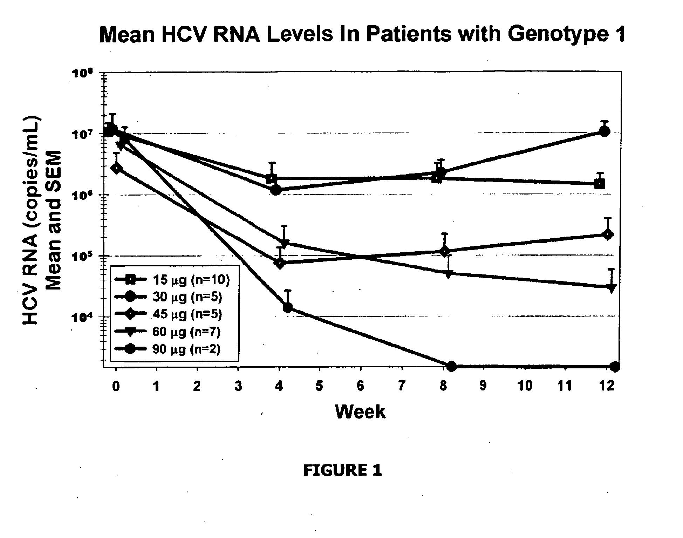

Figure 1: This Figure provides a plot showing the relationship between hepatitis C virus (HCV) ribonucleic acid (RNA) levels over time for patients with genotype 1 treated with omega interferon.

### DETAILED DESCRIPTION

### Definitions

The term "interferon alpha" (sometimes referred to as "alfa") or "alpha interferon" or "α-interferon" means the family of highly homologous species-specific proteins (i.e. glycoproteins) that are known in the art and that inhibit viral replication and cellular proliferation and modulate immune response. Typical suitable alpha interferons include recombinant interferon alpha-2b such as Intron-A interferon available from Schering Corporation, Kenilworth, N.J., recombinant interferon alpha-2a such as Roferon interferon available from Hoffmann-La Roche, Nutley, N. J., recombinant interferon alpha-2C such as Berofor alpha 2 interferon available from Boehringer Ingelheim Pharmaceutical, Inc., Ridgefield, Conn., interferon alpha-n1, a purified blend of natural alpha interferons such as Sumiferon available from Sumitomo, Japan or as Wellferon interferon alpha-n1 (INS) available from the Glaxo-Wellcome Ltd., London, Great Britain, or a consensus alpha interferon such as those described in U.S. Pat. Nos. 4,897,471 and 4,695,623 and the specific product available from Amgen, Inc., Newbury Park, Calif., or interferon alpha-n3, a mixture of natural alpha interferons made by Interferon Sciences and available from the Purdue Frederick Co., Norwalk, Conn., under the Alferon Tradename.

The term "interferon beta" or "beta-interferon" or "β-IFN" means the proteins (i.e. glycoproteins) as known in the art that have the ability to induce resistance to viral antigens. Examples are described in U.S. Pat. No. 4,820,638 and 5,795,779, and include equivalents or derivatives thereof.

The term "interferon gamma" or "gamma interferon" or γ-IFN" means the proteins (i.e. glycoproteins) that have the ability to induce resistance to certain viral antigens and are described in U.S. Pat. Nos. 4,727,138; 4,762,791; 4,845,196; 4,929,554; 5,005,689; 5,574,137; 5,602,010; and 5,690,925, or equivalents or derivatives thereof.

The term "interferon tau" or "tau interferon" or "τ-IFN" means the proteins (i.e. glycoproteins) that have the ability to induce resistance to certain viral antigens and are described in U.S. Pat. Nos. 5,939,286; and 6,204,022, or equivalents or derivatives thereof.

The term omega interferon or ω-interferon as used herein means the species-specific protein (i.e. glycoprotein) that is described in U.S. Pat. Nos. 5,120,832 and 5,231,176. It can inhibit viral replication, cellular proliferation, and modulate immune response, even in settings or patients where alpha interferon is not effective or has limited effectiveness. Omega interferon is a naturally occurring interferon which has limited homology to the alpha interferons (about 65%) and even less homology to the beta interferons (about 35%). Therefore, omega interferon is structurally distinctive. Nevertheless, as noted above, omega interferon appears to bind to the "α-β receptor" as judged by in vitro testing. Using genetic engineering techniques, recombinant omega interferon is prepared in mammalian cells. We have found that antibodies developing in animals exposed to alpha interferon do not cross react with omega interferon, i.e., that omega interferon is immunologically distinctive.

Throughout the specification and claims, IFN and interferon are used interchangeably.

A non-omega IFN refers to an IFN that is not omega IFN or a combination of IFNs that are not omega IFN. A non-omega interferon would include alpha IFN, beta IFN, gamma IFN, tau IFN, leukoctye-derlved IFN, and the like.

### Method of Treatment

One aspect of this invention is omega interferon for use in a method of treating an infectious disease in a warm-blooded animal subject according to the claims. Omega IFN is administered at a dosage and activity for the disease state treated sufficient to induce a therapeutic response in the animal. Surprisingly, the administered dosage and activity for the disease state treated is higher for omega IFN than would be well-tolerated based on data for non-omega IFNs. Generally, the units of activity per microgram (µg) of omega IFN exceed the units of activity per µg of the non-omega IFN by a factor of more than 1 to about 3, preferably by about 2. Thus, when the dosage is given to the animal for at least a month, the side-effects from the omega interferon administration are less than expected from the use of other interferon products. The preferred dosage of omega interferon is about 135-700 µg/week and the omega IFN activity is about 27-420 million international units (MIU).

More specifically, the invention is omega interferon for use in a method of treating interferon-responsive disorders with both greater tolerability and greater efficacy, thereby improving the therapeutic index for treatment of interferon-responsive disorders. Thus, omega interferon is administered at a dosage and for a period of time sufficient to effect the desired therapeutic response while simultaneously advantageously limiting the undesirable adverse side effects. The omega interferon is administered in combination with one or more adjunctive therapeutic agents, as defined in the claims.

The method may be used in any warm-blooded animal that has not received prior treatment with an interferon and is also useful in treating any warm-blooded animal that shows either residual sensitivity or resistance to treatment using another interferon where either: (1) adverse side effects were unacceptably high; (2) therapeutic response was unacceptably low; (3) or some combination of (1) and (2), The animals may be livestock, household pets, or preferably, humans. Thus, the method has both veterinary and human medicinal uses. Livestock treatable by this method include horses, cattle, swine, sheep, goats, and the like. Household pets include cats, dogs, rabbits, birds and the like. Preferably, however, the method of the invention has its primary application in the treatment of humans, both male and female, whether young or old.

The diseases treatable by the method of this invention are hepatitis C and yellow fever.

As noted the method is useful for treating a patient who has not been previously treated with an IFN or one who has been shown to have a drug-resistance to other non-omega interferons such as alfa IFN, consensus IFN, tau IFN, beta IFN, gamma IFN, leukocyte-derived IFN, and the like. Such resistance may be "primary resistance" to the therapeutic effects of the non-omega interferon, for example, when such interferon is administered alone or when combined with at least one adjunctive therapeutic agent, whether such an agent(s) is used before, during, or after the interferon. The non-omega interferon may be unpegylated, pegylated, or otherwise chemically modified in some manner (e.g., attachment of another protein such as albumin or the attachment of a polyethylene glycol - fatty acid moiety. Such resistance may also be "secondary" to the therapeutic intervention of a non-omega interferon when such interferon is administered alone or in combination with an adjunctive therapeutic agent or agents, again whether such an agent(s) is used before, during, or after the interferon. Such secondary resistance develops during the course of treatment and may be caused by, e.g., anti-interferon antibodies or other cellular or humoral mechanisms that reduce responsiveness to alpha or consensus interferon. Such resistance may be seen with an interferon alone or in combination with an adjunctive therapeutic agent, i,e., an active agent provided with the interferon to supplement or complement the activity of the interferon. Such "resistance" may, in fact, be due only to the failure or inability to administer a therapeutically effective dose of an interferon. The present invention is particularly useful in preventing or eliminating this type of "insufficient-dose resistance."

As part of this invention, it was found that the maximal tolerated dose (MTD) is quite limited for currently available interferons. In a patient with life-threatening cancer, for example, higher MTD's are more acceptable than in a patient with a less aggressive cancer. In patients with chronic hepatitis C, MTD's are lower, sometimes significantly lower. We analyzed the MTD data for many interferons in many different clinical settings (Table 1 below sets forth a collection of representative studies).

**Table 1. Maximal Tolerated Dose Regimens of Interferons Approved for Use in the Treatment of Hepatitis C**

| **#** | **Interferon** | **Indication** | **MTD Regimen** | **Reference** |
|---|---|---|---|---|
| 1 | alfa-2a | hepatitis C | 6 MIU TIW sc | FDA Summary Basis of Approval (PLA 94-0782, 29 Oct 1996) |
| 2 | alfa-2b | hepatitis C | 3-5 MIU TIW sc | FDA approved product labeling |
| 3 | PEG alfa-2b | hepatitis C | 105 µg* QW sc (≈ 21 MIU QW sc) | FDA approved product labeling |
| 4 | PEG alfa-2c | hepatitis C | 180 µg QW sc (≈ 36 MIU QW sc) | Product labeling |
| 5 | alfa-2a | cancer | 30 MIU/wk (+etretrinate) | Roth et al. Acta Oncol 1999; 38(5):613-7 |
| 6 | alfa-2a | cancer | 15.5 MIU/wk over 7 wks | Rajkumar SV et al Int J Radiat Oncol Biol Phys 1998 Jan 15;40(2):297-302 |
| 7 | alfa-2a | cancer | 6 MIU TIW sc (idarubicin, dexamethasone) | Hubel K et al. Leukemia 1997 Dec; 11 Suppl 5:S47-51 |
| 8 | alfa-2a | cancer | 5 MIU TIW sc (all-trans retinoicacid) | Adamson PC et al. J Clin Oncol 1997 Nov;15(11):3330- |
| 9 | alfa-2a | cancer | 3 MIU QD x 7d x 4 wks (+IL2) | Gause BL et al. J Clin Oncol 1996 Aug;14(8):2234-41 |
| 10 | alfa-2a | cancer | 3.4 MIU TIW (+5-FU+cisplatin) | Trudeau M et al. Cancer Chemother Pharmacol 1995;35(6):496-500 |
| 11 | alfa-2a | cancer | 3.4 MIU QDx5d 1 week out of 4 (+RT, + cisplatinum, + hydroxyurea) | Vokes EE et al. Cancer Chemother Pharmacol 1995;35(4):304-12 |
| 12 | alfa-2a | Cancer | 3 MIU TIW (+cisplatinum) | Gosland MP et al. Cancer Chemother Pharmacol 1995;37(1-2):39-46 |
| 13 | alfa-2b | Cancer | 3.6 MIU QD sc | Dorr RT et al J Interferon Res 1988;8:717-25 |
| 14 | alfa-2b | Cancer | 24 MIU/m² QD iv limited to 7 days; max 12 MIU/m2 QD over two weeks (one patient) | Iacobelli S et al Am J Clin Oncol 1995; 18:27-33 |
| 15 | alfa-2b | Cancer | 5 MIU/m² TIW sc | Kirkwood JM et al J Clin Oncol 1996;14:7-17 |
| 16 | PEG alfa-2b | Cancer | 7.5 µg/kg QW (35 MIU/wk) | Talpaz M et al, Blood 2001;98:1708-13 |
| 17 | consensus | Hepatitis C | 3-9 µg** TIW sc (≈ 3-9 MIU TIW sc) | FDA approved product labeling http://www.fda.gov/cber/sba/ifn amg100697S.pdf |

| | | | | |
|---|---|---|---|---|
| Footnotes to table: * assumes that pegylated IFN is as potent µg per µg as unpegylated; ** assumes that consensus is about 5 x as potent as alfa-2a or -2b | | | | |

Using the same study designations as above, the maximal tolerated dose per month calculated in MIU, for each dosing regimen, is shown below. Adjustments for weight or body surface area were made to normalize doses.

It should be noted that in Table 1 that MIU represents millions of international units of antiviral activity; in some instances there is a µg-equivalent. The relative potency of interferons also needs to be considered, however. For example, although the clinical activity profile of pegylated alfa interferon tends to be better in some instances than that of unpegylated alfa interferon (because the pegylation improves pharmacokinetics, e.g., by keeping the molecule in circulation longer), there is a loss in antiviral activity on a µg for µg basis when pegylated interferon is directly compared to unpegylated alfa interferon. Pegylation improves pharmacokinetics at the expense of (in vitro) antiviral activity. In general, we observed that for all pegylated or all unpegylated interferons, MIU of antiviral activity is also an excellent surrogate or predictor of side effects. The higher the MIU, the greater the rate and severity of side effects and the lower the MTD and the lower the achievable efficacy.

**Table 2. Maximal Tolerated Interferon Dose Over 4 Weeks**

| **#** | **Interferon** | **Indication** | **Maximal Tolerated 4-Week Dose (MIU)** |
|---|---|---|---|
| 1 | alfa-2a | hepatitis C | 72 |
| 2 | alfa-2b | hepatitis C | 60 |
| 3 | PEG alfa-2b | hepatitis C | 34 or 84* |
| 4 | PEG alfa-2a | hepatitis C | 36 or 144* |
| 5 | alfa-2a | cancer | 120 |
| 6 | alfa-2a | cancer | 62 |
| 7 | alfa-2a | cancer | 72 |
| 8 | alfa-2a | cancer | 20 |
| 9 | alfa-2a | cancer | 84 |
| 10 | alfa-2a | cancer | 41 |
| 11 | alfa-2a | cancer | 68 |
| 12 | alfa-2a | cancer | 36 |
| 13 | alfa-2b | cancer | 100 |
| 14 | alfa-2b | cancer | 290 |
| 15 | alfa-2b | cancer | 60 |
| 16 | PEG alfa-2b | cancer | 140 |
| 17 | consensus | hepatitis C | 36 - 108 |

| | | | |
|---|---|---|---|
| * dependent upon the presumed or calculated ratio of MIU/µg, as discussed herein before. | | | |

The MTD's derived from FDA-approved product labeling involved multiple phase I, II, and/or III clinical trials for each of the designated interferons. Other references represent single studies.

We have observed that it is common error to overestimate MTD's on the basis of small sample or cohort sizes, particularly in phase I studies. A good example is the recent experience with pegylated interferon alfa-2a. A 27 patient study with 3-6 patients per dosing group initially concluded that 450 µg was a suitable weekly dose [see Motzer RJ et al J Clin Oncol 2001;19:1312-9]. (With an estimated antiviral activity, however, of only 7% of the unpegylated interferon, 450 µg pegylated alfa-2a provided only 70% of the antiviral activity of 45 µg regular alfa-2a as measured by in vitro assays (see Bailon p et al Bioconjug Chem 2001;12:195-202.) Despite these optimistic estimates, subsequent larger studies have generally been limited to doses of 180 µg per week or less (PEGINTERFERON ALFA-2a IN PATIENTS WITH CHRONIC HEPATITIS C. Zeuzem S, Feinman SV, Rasenack J et al. New Engl J Med 2000;343:1666-72).

As may be seen by an inspection of Table 2, however, the maximal tolerated dose for any interferon approved for the treatment of hepatitis is no more than 180 MIU over 4 weeks. This maximum holds even with the most favorable assumptions regarding the number of antiviral MIU per µg of interferon administered. For alfa-2a, alfa-2b, and consensus interferon, doses above the indicated maximum yielded unacceptable, severe, sometimes irreversible clinical toxicities or caused patients to stop treatment.

The potencies of the various interferons identified previously are measured by different assay systems, yielding various ratios of MIU/µg. As a result, the cumulative and mean ± SD MIU values for various interferons may differ based on the presumed MIU/µg of a given interferon. Such assumptions are accommodated in the calculations shown below (Table 3).

**Table 3. Average 4-Week Doses for Hepatitis and Cancer**

| **Scenario** | **Indication** | **Study Numbers** | **Potency Assumptions** **(Ratio of MIU/µg)** | **Maximal Average Tolerated 4-Week Dose (MIU)** **Mean ± SD** |
|---|---|---|---|---|
| 1 | Hepatitis | 1-4 | 3 MIU ≈ 15 µg for alfa-2a and alfa-2b, pegylated or unpegylated interferon of equal MIU/µg | 90 ± 37 |
| 2 | Hepatitis | 1-4 | Pegylated interferon ≈ 40% as potent as unpegylated interferon, µg per µg in vitro | 51 ± 19 |
| 3 | Hepatitis | 1-4, 17 | Same as 1 and consensus interferon potency ≈ 5 x potency alfa-2a or alfa-2b | 108 ± 52 |
| 4 | Hepatitis | 1-4, 17 | Same as 2 and consensus interferon potency ≈ 1 MIU/µg | 48 ± 17 |
| 5 | Cancer | 5-16 | 3 MIU ≈ 15 µg | 94 ± 33 |
| 6 | | | | |

The "best tolerated" average MIU continuous MIU exposure over at least 4 weeks would appear to be that represented by the calculations shown in Scenario 3 above, which assumes a very high ratio of MIU to µg for the study of consensus interferon. The mean ± SD = 108 ± 52 while the mean ± SEM would be 108 ± 26. Accordingly, any MIU/4-week value in excess of the mean + 3 SEM's, or 186, would be unexpected for all interferons considered together. None of the referenced regimens meets this test, even if it is assumed that the µg/µg potency of pegylated interferon is the same as the matching unpegylated interferon. In general, in vitro testing does not substantiate this assumption (see Bailon P reference above).

Once the data is presented in this fashion, it is clear that there is a significant need for an interferon having a more favorable efficacy/side effect profile. It is desirable that the interferon be administered at higher doses to achieve greater antiviral effects with still-acceptable clinical tolerability.

In the setting of treating hepatitis C or cancer, some side effects may be worsened by the addition of ribavirin (hepatitis), interleukin-2 (cancer), or other adjunctive therapies now in use or under development (cell pathway blockers such as, for example, tyrosine kinase inhibitors).

Inadequate treatment of hepatitis, cancer or other interferon-responsive disorders can also occur because of highly variable levels of interferons caused by intrinsic biological variability in patients. Perhaps more importantly, however, we have observed that interferon blood levels are quite variable for interferons such as alpha, consensus, and potentially tau, because of short half-lives in blood. Such variation in blood levels is important when dosing of alpha interferon is given, for example, daily (QD), every other day (QOD), three times weekly (TIW), or once weekly (QW). Important variability in blood levels occurs even when modified interferons such as pegylated alpha interferon are administered QW. Such variability may contribute further to the occurrence of and unpredictable nature of adverse side effects in patients with, for example, chronic hepatitis C.

There is a clear medical need for a safer, better tolerated, and more effective interferon with antiviral, immunomodulatory, and/or antiproliferative properties. In particular, in the treatment of hepatitis C, there is a need for an interferon that can be administered at higher doses with greater tolerability and a lower incidence and lesser severity of adverse side effects, i.e., an interferon with a superior therapeutic index.

In the therapeutic area of hepatitis, for example, there is also a need for an interferon with an improved pharmacokinetic profile which is active as monotherapy or as part of combination therapy in patients for whom alpha interferon alone or in combination with, e.g., ribavirin is judged to be inadequate treatment, especially for patients infected with one or more hepatitis C viruses or viral subtype(s) that are partially or wholly resistant to therapy with, e.g., an alpha or a consensus interferon.

In addition, there is a need for effective and safe interferon therapy which is capable of safely and tolerably suppressing viral replication to acceptable levels for months or even years if complete eradication cannot be achieved.

Surprisingly, we have now discovered that omega interferon is not only effective in treating patients chronically virally infected, e.g., with hepatitis C virus but is particularly well tolerated. Moreover, omega interferon is tolerated at µg- and MIU-dose levels that are much higher than those that can be safely used with other interferons such as alpha or consensus interferons, by way of examples. This clinical tolerability obtains even though omega interferon binds to the receptor to which the alpha interferons and consensus interferon also bind. In addition, this surprising effectiveness and tolerability occurs even though omega interferon has a significantly greater potency (MIU/µg) than the alpha interferons and thus would be predicted to be unusable at higher doses.

Thus, we discovered that in the disease state treated, the dosage and activity of omega IFN was sufficient to induce the desired therapeutic response in the animal without the predicted poor tolerance of such treatments using a non-omega IFN. This gives a physician far greater flexibility in the treatment of different diseases. For example, in treating hepatitis C, higher doses of omega IFN may be needed as compared to the potentially lower doses needed for hepatitis cirrhosis. In either case, however, a higher and more efficacious yet better tolerated dose of omega IFN could be used to treat the disease than one of skill in the art would expect from current data for non-omega IFNs.

As one of several favorable clinical consequences of the safety and tolerability profile, we have also demonstrated that omega interferon can suppress hepatitis C viral replication in the most interferon-resistant viral subtype, namely HCV genotype 1. Equally surprising, we have also discovered that, at appropriately chosen dose levels, unpegylated omega interferon alone suppresses hepatitis C viral replication in genotype 1 more effectively than other available therapies. This superiority for unpegylated omega interferon is evident when current clinical results with omega interferon are compared with historical clinical data from studies of:
1. other unpegylated interferons administered alone;
2. pegylated interferons administered alone; and, most surprisingly
3. the expensive and toxic two-drug antiviral regimen of an alpha interferon plus ribavirin.

Moreover, it has also been demonstrated in vitro In cells infected with the immunodeficiency virus that the patterns of gene signaling induced by alpha and omega interferon are different, i.e., the omega interferon is functionally distinctive. Omega interferon is able to induce more sustained anti-HIV gene responses when the responses to alpha interferon are transient. We have also now demonstrated that omega interferon is uniquely able to substantially suppress viral replication of, e.g., the yellow fever virus when other Interferons cannot.

In the method of this invention, the omega IFN is administered in conjunction with an adjunctive therapeutic agent, i.e., a physiologically or pharmacologically active material that complements or supplements the activity of the omega IFN, being an inosine monophosphate dehydrogenase inhibitor (IMPDI) such as ribavirin or a ribavirin analog is often used. Other inosine monophosphate dehydrogenase inhibitors include mycophenolic acid, mycophenolate mofetil, mycophenolic acid sodium, aminothiadiazole, thiophenfurin, tiazofurin, viramidine, VX-148, VX-497, and VX-944. Other non-IMPDI agents include interleukln-2 or an interleukin-2 derivative, histamine, a histamine derivative, a monoclonal antibody, a polyclonal antibody, or a small molecule inhibitor of hepatitis C viral replication. Specific examples of such antibodies include HBV-Ab (XTL)-17 and -19.

Ribavirin is known chemically as 1-β-D-ribofuranosyl-1H-1,2,4,-trlazole-3-carboxamide and is available from ICN Pharmaceuticals, Inc., Costa Mesa, California. It is described in the Merck Index, 11^{th} Edition at 8199. Its manufacture and formulation is described in U.S. Pat. No. 4,211,771.

VX-497 is known chemically as (S)-N-3[3-(3-Methoxy-4-oxazol-5-yl-phenyl)-ureido]-benzyl-carbanic acid tetrahydrofuran-3-yl ester and is available from Vertex Pharmaceuticals, Inc., Cambridge, Massachusetts. It is further described in Pharmaprojects® and in U.S. Pat. No. 5,807,876.

Mycophenolic acid in known chemically as 6-(1,3-dihydro-4-hydroxy-6-methoxy-7-methyl-3-oxo-5-isobenzylfluranyl)-4-methyl-4-hexanoic acid and is produced by Penicillium brevi-compactum, P-stoloneferum, and related spp. It is further described in the Merck Index, Eleventh Edition at 6238.

Mycophenolic acid-sodium is the sodium salt of mycophenolic acid and is available from Novartis Corp., Basel, Switzerland.

Mycophenolate mofetil is the 2-morpholinoethyl ester of mycophenolic acid and is available as CellCept® from Roche Laboratories, Inc., Nutley, New Jersey. It is further described in the Physicians Desk Reference, 53^{rd} Edition at p. 2657.

Aminothiadiazole is 1,3, 4-thiadiozol-1-amine and has the CAS Registry No. 4005-51-0. The molecular weight is 101.004755 and the molecular formula is C2H3N35. Further information is available from the service Pharmaprojects, Accession No. 5433.

Thiophenfurin and tiazofurin are compounds that have *in vivo* activity in mice. See J. Med. Chem., 1995, 38, 3829 and Pharmaprojects.

Viramidine is a ribavirin derivative to be used a monotherapy or in combination with an IFN for hepatitis C. ICN Pharmaceuticals is pursuing the compound. Also see Pharmaprojects.

VX-148 and VX-944 are being developed by Vertex Pharmaceuticals. See Pharmaprojects for further information.

CDN-4007 is a compound originated by Oncor, Inc. Further information may be found in Pharmaprojects, Accession No. 25549.

XTL-17 and XTL-19 are monoclonal antibodies directed to hepatitis C.

Once a patient who has a suspected interferon-sensitive disorder is identified, the patient is then treated by administering an amount of omega interferon with an adjunctive therapeutic agent as defined in the claims, for a time sufficient to effect a therapeutic response while mitigating any adverse side effects of therapy. The amount of omega interferon will be determined by the doctor administering the dose on a patient-by-patient basis depending on factors such as, by way of example age, body weight and habitus, gender, concomitant medical disorders, concomitant medications, known or suspected genetic profile etc.

To enhance therapeutic response, the amount of omega interferon will preferably be greater than the amount (judged by mass or potency as appropriate) employed using a different interferon. If a patient with a disease or condition resistant to prior therapy with another interferon is identified, a similar process is followed.

In a preferred embodiment, omega interferon is administered parenterally (i.e., by injection not to the gut, e.g., intramuscularly, intraperitoneally, intravenously, or subcutaneously) to a human patient with an interferon-responsive disorder in a dose of about 135-700 µg per week or about 19-420 MIU per week. The dose may be administered by single injections of small amounts, for example 15-100 µg per dose. Such amounts may be administered continuously, multiple times per day, QD, QOD, TIW, or QW. Such amounts or larger amounts may be administered also by depot or sustained release formulations, e.g., containing 270-10,000 µg. Such depot administrations or sustained release forms are given less frequently than once a week and are intended to remain in the body for at least two weeks or even more than a month. For example, a 12-week dosage for controlled release at a relatively constant rate of 175 µg per week (representing about 35-105 MIU/week) would have about 2100 µg of omega IFN (12 x 175), while a 24-week dosage would have 4200 µg (24 x 175), etc. All appropriate dosage forms and routes of administration may be utilized.

The method is particularly useful for treating a human patient with chronic hepatitis C.

In another embodiment, omega interferon is administered enterally, especially orally. Such administration may occur as a single dose or multiple doses during the week. Omega interferon may be administered in essentially pure form or mixed with one or more excipients and may be chemically or physically modified to enhance bioavailability.

For example, in addition to parenteral administration (e.g., intravenously, intramuscularly, intraperitoneally) omega interferon may be given topically or by inhalation. Effective dosing with omega interferon may also be achieved by increasing endogenous omega interferon, or a fragment thereof, by the use of specific or non-specific inducers or by the administration of genetic material (such as nucleic acid(s)) which encodes all of or part of the genetic material required to express omega interferon.

Pharmaceutical formulations comprising omega interferon may also comprise at least one pharmaceutically acceptable carrier, which may include excipients such as stabilizers (to promote long term storage), emulsifiers, binding agents, thickening agents, salts, preservatives, solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the omega interferon, its use in the therapeutic compositions and preparations is contemplated. Alternatively, cells expressing omega interferon may also be administered, preferably producing amounts of omega interferon sufficient to increase effectiveness without materially increasing adverse side effects when compared to other interferons. Moreover, omega interferon may be administered by non-cellular delivery systems such as liposomes.

Because the treatment of proliferative, immunologic, or infectious diseases generally is carried out over longer periods of time, it may in such cases be preferable to implant or inject an article having a formulation of omega IFN that is biocompatible for the subject being treated and releases the IFN is a regulated manner over time, i.e., a controlled-release formulation. The formulation may be bioerodible, e.g., a gel, nongel polymer, or pellet, or nonbioerodible, e.g., a mechanical device such as a pump. A pump may also be external to the body proper with only a catheter or tube or the like penetrating the skin into a subcutaneous or intramuscular space.

An example of a suitable nonbioerodible formulation or device is one employing the DUROS® system (ALZA Corporation), which is a miniature drug-dispensing pump currently made principally from titanium and which can be as small as a wooden matchstick.

The DUROS® pump operates like a miniature syringe loaded with a drug inside the drug reservoir. Through osmosis, water from the body is slowly drawn through a semipermeable membrane into the pump by a salt or other suitable osmotically active substance residing in the engine compartment. This water is absorbed by the osmotic substance which then swells and which slowly and continuously pushes a piston, dispensing the correct amount of drug out the drug reservoir and into the body. The osmotic engine does not require batteries, switches or other electromechanical parts in order to operate. The amount of drug delivered by the system is regulated by many factors, including, for example, the materials used in manufacturing, the membrane's control over the amount of water entering the pump, the strength of the osmotic agent, the frictional resistance to motion of the piston, the size and shape of the reservoir, the size, shape, and length of the orifice(s) through which the drug(s) exit the pump, the formulation and type of the drug(s) and whether the formulation is a liquid, suspension, or gel, and pressures generated within the device to expel drug(s) or counter-pressures generated in the tissues that resist such expulsion.

Other useful long-term delivery formulations may be prepared using the ALZET® technology developed by the ALZA Corporation. These formulations may be delivered externally. The details of the ALZET technology may be found at www.alzet.com.

Patents that provide useful guidance in preparing long-term delivery devices that may be useful in the methods and kits of this invention include those which are assigned to Alkermes. Other patents include those assigned to ALZA Corporation (now a subsidiary of Johnson and Johnson, Inc.), particularly relating to their "DUROS®" technology. Representative patents useful for the various aspects of this invention include the following U.S. patents: 5,529,914; 5,858,746; 6,113,938; 6,129,761; 5,985,305; 5,728,396; 5,660,847; 5,112,614; 5,543,156; 5,443,459; 5,413,572; 5,368,863; 5,324,280; 5,318,558; 5,221,278; 4,976,966; 4,917,895; and 4,915,954.

When an adjuvant therapeutic agent such as ribavirin (or other IMPDI) is administered, ribavirin is administered to the patient in association with omega interferon that is, the ribavirin dose is administered during some or all of the same period of time that the patient receives omega interferon. Most Interferon formulations are not effective when administered orally except when chemically modified as described above or protected in some other manner from degradation by gut peptidases. Accordingly, the preferred method of administering the omega interferon is parenterally, preferably by subcutaneous, intravenous, or intramuscular, Injection. More preferable would be the administration of a depot form, with or without the use of a device such as a pump, or the administration of another long-term dosing form suitable for multiweek or multimonth, continuous or continual delivery of omega Interferon. The pump may be of any suitable design such as a fixed or variable delivery osmotic, electrical, mechanical, hydraulic, gas-powered and inserted beneath the skin or worn externally.

The IMPDI, e.g., ribavirin may be administered orally in capsule or tablet form in association with the administration of omega interferon. Of course, other types of administration of both medicaments, as they become available are contemplated, such as by nasal spray, transdermally, by suppository, by sustained release dosage form, etc. Any form of administration will work so long as the proper dosages are delivered without destroying the active ingredient and proper consideration is given to the individual absorption, distribution, metabolism, and excretion of the combination at various dose levels.

Ribavirin is generally administered at the rate recommended by the Physicians Desk Reference© , but may be administered at a rate of about 400 to about 1200 mg/day.

Another aspect of the invention can be viewed as omega interferon for use in a method wherein the omega interferon is administered to a subject in need thereof in combination with a therapeutically effective amount of at least one adjunctive therapeutic agent as defined in the claims, for as long a period of time as the animal tolerates omega interferon, monitoring the levels of a disease marker In the animal during the administration, and continuing the administration omega interferon for so long as the levels of the disease marker continue to be reduced.

The course of the disease and adverse effects, if any, are then monitored by the doctor caring for the patient. This may be done by evaluating the signs and symptoms of the disease or by monitoring the patient's fluids (e.g., blood, plasma, urine) for the presence of such a disease marker. For example, a person suffering from chronic hepatitis C virus ("HCV") infection may exhibit one or more of the following signs or symptoms:
(a) elevated alanine aminotransferase ("ALT"),
(b) elevated aspartate aminotransferase ("AST")
(c) elevated bilirubin
(d) positive test for anti-HCV antibodies,
(e) presence of HCV as demonstrated by a positive test for the disease marker HCV-RNA,
(f) clinical stigmata of chronic liver disease such as abnormal liver size, asdtes or esophageal varices, and
(g) hepatocellular damage or dysfunction shown by histopathology, laboratory or radiographic means
(h) hepatocellular carcinoma.

In a patient having a severe HCV infection the number of HCV-RNA copies per ml of serum in the patient may exceed 2 x 10⁶ copies, By successful treatment by the method of this invention the number of copies of HCV-RNA may be reduced to levels that a nearly undetectable, i.e., below about 100 -1000 copies of HCV-RNA per ml of patient serum as measured by quantitative, multicycle, reverse transcriptase PCR methodology.

Thus, it can be seen that another aspect of this invention can be viewed as the use omega interferon (IFN) in the manufacture of a medicament for treating an infectious disease In a warm-blooded animal as defined in the claims. The medicament is for administration to the animal In accordance with the teachings for hereinbefore, i.e. at a dosage and activity for the disease treated sufficient to induce a therapeutic response in the animal, which dosage and activity for the disease state treated is higher than would be well-tolerated based on data for non-omega IFNs. The preferred aspects of the method of treatment would also apply to this "use" aspect of the invention.

### Article of Manufacture

Described herein is an article of manufacture useful for treating an immunologic, proliferative, or infectious disease in a warm-blooded animal subject. The article comprises omega interferon suitable for administering a therapeutically effective amount of the omega IFN to the subject in combination with dosing instructions for administering the omega IFN at a dosage that is higher than would be predicted based on data for a non-omega IFN, wherein the dosage is preferably given to the animal for at least a month and wherein the side-effects from the omega interferon administration are less than expected from the use of other interferon products. The article is suitable for enteral, parenteral, inhalation, or topical administration as discussed hereinabove Preferably, the article is designed for Injection into the subject, particularly for subcutaneous injection. The article is particularly valuable designed to administer the omega IFN in a controlled release manner into the subject, e.g., at a rate of about 135-700 µg per week (representing about 27-280 MIU per week). Extending the controlled rate for at least one month is preferred. Of course, if the omega IFN is formulated for injections, it is preferably a sterile aqueous composition,

Also described herein is a kit useful for delivery of a relatively constant amount of a drug thereof over time, wherein the amount of drug delivered to an individual patient is about 135-700 µg/week. The kit comprises a long-term delivery formulation designed for delivery of a drug at a relatively constant rate over time, generally at least one month, preferably 3-12 months. The kit may also comprise other devices or medicaments useful in the administration of the formulation. The doctor or other provider of health care can Individualize the dosage rate for a patient over time depending on the patient's characteristics such as age, gender, size, health condition, etc. and the severity and type of disease.

### Process of Manufacture

Described herein is a process for preparing an omega IFN-based article of manufacture useful for treating an immunologic, proliferative, or infectious disease in a warm-blooded animal subject. The process comprises providing omega IFN as a composition suitable for administering to the subject at a therapeutically effective dosage, and combining the omega IFN so provided with instructions for administering the omega IFN for such disease at a dosage that is higher than would be predicted based on data for a non-omega IFN wherein the side-effects from the omega IFN administration are less than expected from the use of other interferon products. Such a process will result in the omega IFN being suitable for enteral, parenteral, or topical administration, preferably for injection into the subject. The process resulting in the omega IFN being suitable for subcutaneous injection, especially for controlled release of the omega IFN into the subject at a rate of about 135-700 µg (27-280 MIU) per week is preferred. The controlled rate of release can extend for one month or more. If the process is designed to formulate omega IFN as a composition for injection, it is important that it is sterile, preferably as a sterile, aqueous solution.

Also described herein is a method of manufacturing a delivery system for delivering omega IFN over time in a controlled manner. The method comprises preparing a long-term delivery device designed for delivery of an omega IFN at a relatively constant rate over time, the rate being determined to be about 135-700 µg/week for a patient to receive a dosage amount to treat a disease state in the patient. Once the system is prepared, it is combined with the appropriate written instructions for administration to a subject in need thereof, as discussed hereinbefore. The system may also be combined with other devices or medicaments useful in the administration or delivery of the system. The written dosing instructions can be applied directly to a container (such as by the application of a label directly to a vial containing the interferon with or without carriers or exclplents). Alternatively, a container-closure system holding the interferon can be placed into a second container, such as a box, and the written material, in the form of a packaging insert, can be placed in the second container together with the first container-closure system holding the interferon. The written instructions may describe the indications for prescribing the omega interferon, either as monotherapy or as part of combination therapy with an adjunctive therapeutic agent. Such indications would include an interferon-responslve disorder (for example, viral hepatitis C). The written material would preferably be provided in the form required by the regulatory agency with jurisdiction over the approval for marketing of such an interferon, such as the United States Food and Drug Administration, in the form of a package insert for a prescription drug. The written material would indicate that the interferon would be prescribed for use in patients having infectious, proliferative, or immunologic disease. The written material would preferably describe the technique for administering the drug, e.g., injecting or implanting a formulation. The written material would also contain instructions on the use of the other devices or medicaments contained within the kit. The written material may indicate that the omega interferon is for treating viral hepatitis, in particular viral hepatitis C, or cirrhosis or fibrosis of any organ, in particular the liver when such cirrhosis or fibrosis is caused by viral hepatitis C. The written material would indicate that the interferon is useful as primary or secondary treatment or in combination with other treatments. It would further describe that while the interferon has an effect on the infected liver in patients with viral hepatitis C that the interferon also may reach other tissues where it may have no therapeutic effect or adverse side effects.

Principal toxicities could also be described if appropriate and could include, by way of example, headache, flu-like symptoms, pain, fever, asthenia, chills, infection, abdominal pain, chest pain, injection site reaction (as appropriate), malaise, hypersensitivity reaction, syncope, vasodilatation, hypotension, nausea, constipation, diarrhea, dyspepsia, anorexia, anemia, thrombocytopenla, leukopenia, other blood dyscraslas, myalgla, arthralgia, insomnia, dizziness, suicidal ideation, depression, impaired ability to concentrate mentally, amnesia, confusion, irritability, anxiety, nervousness, decreased libido, urticaria, alopecia, and others.

It may further be described in the written material that when symptoms such as fever, chills, or flu-like manifestations are observed that these can be treated with Tylenol®, antihistamines such as Benadryl®, and that hypotension may respond to the administration of fluids or pressor agents or, if the symptoms or signs are sufficiently severe, that the dose should be reduced or treatment terminated.

The written material may also describe that delivery of the formulation of the interferon intended for short-term administration is by injection, infusion, inhalation, oral or transdermal administration. The preferred embodiment is by injection or infusion and the most preferred is by injection. Warnings, precautions, and contraindications should be described.

### Examples

The following examples are provided for further guidance of how to make and use the invention. In the examples the principal measure of antiviral efficacy in the setting of chronic hepatitis C is the measurement of viral burden for which hepatitis C viral RNA (HCV RNA) is the standard measure. This measurement was utilized in two clinical studies of omega interferon.

In the setting of treating hepatitis, it is useful to measure changes in the following:
(a) elevated ALT
(b) elevated AST
(c) elevated bilirubin
(d) positive test for HBsAg, anti-HBc antibody, anti-HBe antibody
(e) clinical stigmata of chronic liver disease
(f) hepatocellular damage or dysfunction shown by histopathology, laboratory or radiographic means
(g) hepatocellular carcinoma

In the setting of combination therapy with interleukin-2 for the treatment of renal cell carcinoma, it is useful to determine the extent and change in metastatic disease by:
(a) positive computerized tomographic or magnetic resonance imaging scanning
(b) positive bone scan
(c) positive signs on physical examination such as the presence of a palpable mass
(d) positive urinary test for blood.

In the setting of combination therapy with interleukin-2 for the treatment of renal cell carcinoma it is useful to determine the extent and change in metastatic disease by:
(a) positive computerized tomographic or magnetic resonance imaging scanning
(b) positive bone scan
(c) positive signs on physical examination such as the presence of a palpable mass

### Example 1

The safety, tolerability, and antiviral effect of omega interferon was studied in 90 previously untreated patients who were chronically infected with hepatitis C virus of genotype 1, 2, 3, or 4. Other causes of liver dysfunction were excluded. The minimal HCV RNA level at admission was > 100,000 U/mL associated with an elevated level of ALT.

The aims of this study were to evaluate the effect of different doses of omega interferon on HCV RNA levels, alanine aminotransferase (ALT) levels. Further aims were to assess the safety and tolerability of rising doses of omega interferon as judged by physical examinations, adverse side effects and laboratory examinations.

The study was designed as multi-center, open-label, and escalating dose in five cohorts of 15 or more subjects each: 15, 30, 45, 60, and 90 µg administered subcutaneously three times weekly. Therefore, the weekly doses of omega interferon in the five cohorts were 45, 90, 135, 180, and 270 µg. The cumulative weekly antiviral activities of these same doses of omega interferon were, respectively, 18, 36, 54, 72, 108, and 144 MIU. The cumulative 4-weekly antiviral activities of these same doses of omega interferon were, respectively, approximately 72, 144, 216, 288, and 432 MIU. [The antiviral activity was determined by measuring the antiproliferative effects of omega interferon as compared to those of alfa-2c in human A549 cells infected with an encephalomyocarditis virus. In this assay system, the antiviral activity of omega interferon was approximately 4 x 10⁸ U/mg compared to 2 x 10⁸ U/mg for alfa-2a.]

Doses were administered by a visiting nurse or other medical practitioner to ensure that doses were properly administered, blood tests properly drawn, and adverse side effects identified, recorded, and reported promptly.

Omega interferon was prepared as a stabilized and lyophilized powder and then dissolved in sterile water-for-injection. A dose of 15 µg was initially administered subcutaneously from this preparation on a three-times weekly schedule with target dosing durations of 3-12 months. Doses of omega interferon were escalated thereafter.

Hepatitis C viral RNA levels (HCV RNA) were measured by quantitative multi-cycle reverse transcriptase polymerase chain reaction technology (Amplicor®, Hoffmann La Roche). HCV RNA levels were measured three times at least two weeks apart prior to beginning treatment and on treatment days 1-5 and at treatment weeks 2, 4, 8, 12, and 16. If the patient responded to therapy, therapy was continued at the same dose level and HCV RNA levels were measured at quarterly intervals thereafter. Levels of ALT were measured by standard laboratory techniques at pretreatment, day 0, and treatment weeks 2, 4, 8, 12, and 16 and, if treatment continued, quarterly thereafter.

Safety was determined through regular physical examination, regular questioning of patients regarding adverse side effects, and standard laboratory testing that included hematology, chemistry, liver function tests, and the like.

The results of the study are shown below. Baseline characteristics were similar in all dosing groups. The majority of patients were males, most 20-50 years of age. The majority of patients had genotype 1 infection with very high baseline viral loads as measured by HCV RNA. Inflammation of the liver was present as judged by elevated ALT levels that were approximately 3 x normal values.

**Table 4. Baseline Characteristics**

| **Dose/wk (µg)** | **Sex M/F (%)** | **Age (mean ± SD)** | **HCV Genotype** | | **HCV RNA copies/mLx10⁶ (mean ± SD)** | **ALT x Upper Limit of Normal (mean ± SD)** |
|---|---|---|---|---|---|---|
| | | | 1 (%) | 2,3,4 (%) | | |
| 45 | 100/0 | 35 ± 8 | 72 | 28 | 8.7 ± 11.6 | 3.0 ± 1.8 |
| 90 | 72/28 | 40 ± 10 | 61 | 39 | 10.1 ± 16.7 | 2.9 ± 2.7 |
| 135 | 78/22 | 35 ± 7 | 50 | 50 | 7.1 ± 11.1 | 2.4 ± 1.0 |
| 180 | 72/28 | 39 ± 10 | 56 | 44 | 5.8 ± 6.1 | 2.8 ± 1.5 |
| 270 | 100/0 | 31 ± 10 | 34 | 66 | 9.8 ± 13.0 | 2.6 ± 1.4 |
| Total | 84/16 | 36 ± 9 | 55 | 45 | 8.0 ± 11.1 | 2.8 ± 1.7 |

Treatment with omega interferon was surprisingly effective and very well tolerated. The change in HCV RNA levels of viral genotype 1 in treatment groups 1-5 are shown below. At 45, 60, and 90 µg TIW (135, 180, 270 µg/week), there is a clear dose-response at treatment week 12 (Figure 1). There was an excellent virologic response to treatment and a clear dose-response (Table 5). Very surprisingly, in genotype 1 complete viral clearance (below the limits of detectability on the HCV RNA assay) exceeded 80% for the two highest dose groups. Responses in genotypes 2, 3, and 4 were even higher. Resolution of hepatic inflammatory changes, as judged by the biochemical response of changes in ALT levels, were also pronounced, even in patients infected with genotype 1 virus (Table 6).

**Table 5. Virologic Response for Genotype1 at 12 Weeks**

| **Dose (µg/week)** | **Antiviral Activity (MIU/week)** | **Patients with Undetectable HCV RNA (%)** |
|---|---|---|
| 45 | 18 | 20 |
| 90 | 36 | 20 |
| 135 | 54 | 60 |
| 180 | 72 | 82 |
| 270 | 108 | 100 |

Genotypes 2, 3, and 4 responded at even higher overall rates except in the 270 µg/week group where genotype 1 had already achieved the maximum of 100%.

**Table 6. Biochemical Response for Genotype 1 at 12 Weeks**

| **Dose (µg/week)** | **Patients with Normal ALT (%)** |
|---|---|
| 45 | 50 |
| 90 | 50 |
| 135 | 60 |
| 180 | 57 |
| 270 | 100 |

Genotypes 2, 3, and 4 responded at even higher overall rates (except, of course, in the 270 µg/week group where the response rate was already the maximum of 100%).

There were adverse side effects that were mild and temporary or reversible (Table 7). Only one patient was discontinued from dosing because of an adverse event.

**Table 7. Incidence of Adverse Side Effects by Dose**

| | **Percentage (%) of Patients by Weekly Dose** | | | | |
|---|---|---|---|---|---|
| **Adverse Event** | **45 µg** | **90 µg** | **135 µg** | **180 µg** | **270 µg** |
| Influenza like illness | 33 | 63 | 67 | 80 | 100 |
| Leukopenia | 53 | 63 | 42 | 27 | 50 |
| Headache | 67 | 13 | 33 | 40 | 0 |
| Fatigue | 47 | 25 | 8 | 13 | 0 |
| Arthralgia | 53 | 13 | 0 | 13 | 0 |
| Sweating increased | 33 | 13 | 17 | 13 | 0 |
| Injection site reaction | 40 | 0 | 0 | 13 | 0 |
| Myalgia | 33 | 0 | 8 | 0 | 17 |
| Insomnia | 20 | 13 | 17 | 20 | 0 |
| Pyrexia | 27 | 13 | 17 | 7 | 0 |
| Back pain | 7 | 25 | 17 | 0 | 17 |
| Febrile sensation | 20 | 0 | 0 | 13 | 0 |
| Rigors | 13 | 13 | 0 | 13 | 0 |
| Appetite decreased | 20 | 0 | 0 | 13 | 0 |
| Dizziness | 20 | 13 | 0 | 7 | 0 |
| Depression | 0 | 13 | 0 | 27 | 0 |

*Comment.* Historically, alpha interferon alone has been the available therapy in patients with hepatitis C. It is generally recognized that with longer-term treatment, the opportunity for response to treatment rises. The development of the combination of alpha interferon plus oral ribavirin increased response rates. Pegylated alpha interferons may offer advantages over unpegylated alpha interferons as monotherapy. However, it is not clear that pegylated alpha interferon plus ribavirin does not have an increase in adverse side effects that outweigh the benefits of combination therapy.

In recently completed studies, results with an alpha interferon alone have been compared to results with either a pegylated alpha interferon or to the combination of alpha interferon plus oral ribavirin. These results are shown below (Table 8).

**Table 8. Virologic and Biochemical Responses to Non-Omega Interferon Regimens**

| **Study** | **Alpha Interferon** **(6 MIU TIW)** | | **Pegylated Alpha Interferon** **(180 µg/wk)** | | **Alpha Interferon** | | **Alpha Interferon + Ribavirin** | |
|---|---|---|---|---|---|---|---|---|
| | VR (%) | BR (%) | VR (%) | BR (%) | VR (%) | BR (%) | VR (%) | BR (%) |
| Zeuzem S et al | 28 | 39 | 69 | 46 | - | - | - | - |
| Heathcote EJ et al | 12 | 19 | 38 | 34 | - | - | - | - |
| McHutchison JG et al | - | - | - | - | 29 | 24 | 53 | 58 |

VR = virologic response; BR = Biochemical response; " -" = not applicable References:
1. Zeuzem S, New Engl J Med 2000;343:1666-72... a study of non-cirrhotic patients with chronic hepatitis C
2. Heathcote EJ, New Engl J Med 2000;343:1673-80... a study of cirrhotic patients with chronic hepatitis C
3. McHutchison J , New Engl J Med 1998;339:1485-92...a study of non-cirrhotic patients with chronic hepatitis C

In Zeuzem et al, discontinuation because of adverse effects or reduction in dosages occurred in alfa-2a treated patients at 10% and 18%, respectively. For pegylated alfa-2a the corresponding rates were 7% and 19%. In Heathcote et al the rates for alfa-2a were 8% and 14% and for pegylated alfa-2a 13% and 10%, respectively. In McHutchison et al the rates were 9% and 12% for alfa-2a alone and 8% and 13% while for the combination of alfa-2a and ribavirin the rates were 21% and 26%.

Given the magnitude of dosing with omega interferon in the study of Example 1 (much higher than well-tolerated doses with other interferons), the duration of treatment with omega interferon (sufficient for time-dependent serious adverse events to be detected), the virologic response rate (surprisingly high compared even to multidrug therapy with alpha interferon plus ribavirin), it is clear that the virologic response rate, the biochemical response rate, and the tolerability profile of omega interferon are all surprisingly good.

Therefore, monotherapy with omega interferon may simplify the treatment of hepatitis: increasing effectiveness, reducing adverse side effects, reducing the costs associated with diagnosing and treating the side effects, and reducing costs of therapy overall. Combination therapy with, e.g., ribavirin and the higher doses of omega interferon will yield even greater therapeutic results.

### Examples 2

Omega interferon is uniquely active against the yellow fever virus. A CPE (virus-induced cytopathogenic effects)-inhibition assay procedure using vital dye uptake is employed to evaluate compounds for antiviral activity against yellow fever virus strain 17/D in the Vero cells, an African green monkey kidney cell line. Antiviral assays are designed to test six concentrations of each compound in triplicate against the challenge virus, in this instance the yellow fever virus (YFV). Cell controls containing medium alone, virus-infected cell controls containing medium and virus, drug cytotoxicity controls containing medium and each drug concentration, reagent controls containing culture medium only (no cells), and drug colorimetric controls containing drug and medium (no cells) are run simultaneously with the test samples.

The plates are incubated at 37°C in a humidified atmosphere containing 5% CO₂ until maximum CPE is observed in the untreated virus control cultures (Day 6). CPE inhibition by the compound is determined by Cell Titer 96 ®AQueous One Solution Cell Proliferation assay. The assay is a colorimetric method for determining the number of viable cells. The reagent contains a novel tetrazolium compound, MTS [(3-(4,5-dimethylthiazol-2-yl)-5-(3-carboxymethozyphenyl)-2-(4-sulfophe nyl)-2H-tetrazolium, inner salt)], and an electron coupling agent, PMS [phenazine methosulfate]. When combined the MTS and PMS form a stable solution. The MTS tetrazolium compound is then bioreduced into a formazan product by NADPH or NADH produced by dehydrogenase in metabolically active cells. The quantity of formazan product measured is directly proportional to the number of living cells in culture.

A typical arrangement of cells, drugs in variable concentrations, and controls for a standard 8x12, 96-well plates is shown in Tables 9A and 9B. Each table shows one-half of the standard 96-well plate. The tables are presented in this manner only to meet the formatting requirements for this PCT application. The full, standard 96-well plate would be better visualized by placing Table 9A to the left of Table 9B so that there would be 12 columns across the page and 8 rows down the page. The contents of the wells are shown in the various boxes in the tables. In the Tables, various terms have the following meaning:
Media = reagent controls only without cells
Cell control = cells and media
Virus control = Vero grown in the presence of virus but the absence of drug Conc = concentration

**Table 9A**

| | **1** | **2** | **3** | **4** | **5** | **6** |
|---|---|---|---|---|---|---|
| **1** | Media | Media | Media | Media | Media | Media |
| **2** | Cells + Drug 1 conc 1 | Cell Control | Cells + Virus + Drug 1 conc 1 in triplicate | | | Cells + Drug 1 conc 1 |
| **3** | Cells + Drug 1 conc 2 | Cell Control | Cells + Virus + Drug 1 conc 2 in triplicate | | | Cells + Drug 1 conc 2 |
| **4** | Cells + Drug 1 conc 3 | Cell Control | Cells + Virus + Drug 1 conc 3 in triplicate | | | Cells + Drug 1 conc 3 |
| **5** | Cells + Drug 1 conc 4 | Virus Control | Cells + Virus + Drug 1 conc 4 in triplicate | | | Cells + Drug 1 conc 4 |
| **6** | Cells + Drug 1 conc 5 | Virus Control | Cells + Virus + Drug 1 conc 5 in triplicate | | | Cells + Drug 1 conc 5 |
| **7** | Cells + Drug 1 conc 6 | Virus Control | Cells + Virus + Drug 1 conc 6 in triplicate | | | Cells + Drug 1 conc 6 |
| **8** | Drug 1 conc 6 + Media | Drug 1 conc 5 + Media | Drug 1 conc 4 + Media | Drug 1 conc 3 + Media | Drug 1 conc 2 + Media | Drug 1 conc 1 + Media |

**Table 9B**

| | **7** | **8** | **9** | **10** | **11** | **12** |
|---|---|---|---|---|---|---|
| **1** | Blank | Blank | Blank | Blank | Blank | Blank |
| **2** | Cells + Drug 2 conc 1 | Cells + Virus + Drug 2 conc 1 in triplicate | | | Cell Control | Cells + Drug 2 conc 1 |
| **3** | Cells + Drug 2 conc 2 | Cells + Virus + Drug 2 conc 2 in triplicate | | | Cell Control | Cells + Drug 2 conc 2 |
| **4** | Cells + Drug 2 conc 3 | Cells + Virus + Drug 2 conc 3 in triplicate | | | Cell Control | Cells + Drug 2 conc 3 |
| **5** | Cells + Drug 2 conc 4 | Cells + Virus + Drug 2 conc 4 in triplicate | | | Virus Control | Cells + Drug 2 conc 4 |
| **6** | Cells + Drug 2 conc 5 | Cells + Virus + Drug 2 conc 5 in triplicate | | | Virus Control | Cells + Drug 2 conc 5 |
| **7** | Cells + Drug 2 conc 6 | Cells + Virus + Drug 2 conc 6 in triplicate | | | Virus Control | Cells + Drug 2 conc 6 |
| **8** | Drug 2 conc 6 + Media | Drug 2 conc 5 + Media | Drug 2 conc 4 + Media | Drug 2 conc 3 + Media | Drug 2 conc 2 + Media | Drug 2 conc 1 + Media |

The percentage of CPE reduction of the virus-infected wells and the percentage cell viability of uninfected drug control wells are calculated. The minimum inhibitory drug concentration which reduces the CPE by 50% (IC50) and the minimum toxic drug concentration which causes the reduction of viable cells by 50% (TC50) are calculated using a regression analysis program for semilog curve fitting. A therapeutic index (TI50) for each active compound can be determined by dividing the TC50 by the IC50.

The results of the study comparing an alpha interferon (alfa-2b) to omega interferon are shown below (Table 9).

**Table 9. Omega Interferon Halts Replication of the Yellow Fever Virus**

| **Drug** | **IC50 (IU/mL)** | **TC50 (IU/mL)** | **TI (TC50/IC50)** |
|---|---|---|---|
| Alfa-2b Interferon | Not Reached | >200 | NA |
| Omega Interferon | 0.8 | >5000 | >6300 |

Alpha interferon was completely Ineffective against the yellow fever virus. There was no concentration with a measurable antiviral effect. Concentrations above 200 IU/mL produced significant alfa-2b-induced direct cellular injury. As a result it was not possible to calculate a therapeutic index. In contrast, omega interferon produced significant antiviral effects in the absence of drug-induced cytotoxicity and had a TI50 in excess of 6300.

The specific embodiments described herein are offered by way of example only, and the invention is to be limited only by the full disclosure of the specification and the terms of the appended claims, along with the full scope of equivalents to which such claims are entitled.

## Claims

1. Omega interferon, for use in the treatment of infection with a Hepatitis C Virus (HCV) or a yellow fever virus in a warm-blooded animal subject
wherein omega interferon and an adjunctive therapeutic agent is administered to the subject, and wherein the adjunctive therapeutic agent is an inosine monophosphate dehydrogenase inhibitor.

2. Omega interferon for use according to claim 1, wherein the viral disease comprises infection with HCV and the subject exhibits primary or secondary resistance to treatment with a non-omega interferon.

3. Omega interferon for use according to claim 1 or claim 2, wherein the therapeutically effective amount of omega interferon administered in the method is 135-700 micrograms per week.

4. Omega interferon for use according to claim 3, wherein omega interferon activity is about 27-420 million international units (MIU).

5. Omega interferon for use according to any of claims 1-4, wherein the inosine monophosphate dehydrogenase inhibitor is ribavirin or a ribavirin analog.

6. Omega interferon for use according to any of claims 1-4, wherein the inosine monophosphate dehydrogenase inhibitor is selected from the group consisting of mycophenolic acid, mycophenolate mofetil, mycophenolic acid sodium, aminothiadiazole, thiophenfurin, tiazofurin, viramidine, VX-148, VX-497, and VX-944.

7. Omega interferon according to any of claims 5 or 6, wherein the inosine monophosphate dehydrogenase inhibitor administered is administered to a human subject at a therapeutically effective dose of 200 to 4800 milligrams per day.

8. Omega interferon for use according to any of claims 1-7, wherein the omega interferon administered is administered parenterally, enterally, or topically.

9. Omega interferon for use according to claim 8, wherein the omega interferon is administered parenterally.

10. Omega interferon for use according to claim 9, wherein the omega interferon is administered intramuscularly, intraperitoneally, intravenously, or subcutaneously.

11. Omega interferon for use according to claim 10, wherein the omega interferon is administered subcutaneously three times weekly.

12. Omega interferon for use according to claim 9, wherein the omega interferon is administered by injection.

13. Omega interferon for use according to claim 12, wherein the omega interferon is administered by one or more daily injections.

14. Omega interferon for use according to any of claims 1-7, wherein the omega interferon is administered by infusion.

15. Omega interferon for use according to any of claims 1-7, wherein the omega interferon is administered at a controlled rate over time.

16. Omega interferon for use according to claim 15, wherein the omega interferon is administered using a device.

17. Omega interferon for use according to claim 16, wherein the device comprises a pump.

18. Omega interferon for use according to claim 17, wherein the device is an implant in or is external to the subject.

19. Omega interferon for use according to claim 18, wherein the device is an implant in the subject.

20. Omega interferon for use according to claim 19, wherein the device comprises an osmotic pump.

21. Omega interferon for use according to any of claims 1-20, wherein the omega interferon is a recombinant omega interferon.

22. Omega interferon for use according to any of claims 1-21, wherein the subject is a human.

23. Use of Omega interferon and an adjunctive therapeutic agent in the manufacture of a medicament for treating infection with a Hepatitis C Virus (HCV) or a yellow fever virus
, wherein the adjunctive therapeutic agent is an inosine monophosphate dehydrogenase inhibitor.

## Patentansprüche

1. ω-Interferon zur Verwendung bei der Behandlung einer Infektion mit dem Hepatitis-C-Virus (HCV) oder einem Gelbfiebervirus bei einem warmblütigen Individuum,
worin dem Individuum ω-Interferon und ein damit verbundenes therapeutisches Mittel verabreicht werden und worin das verbundene therapeutische Mittel ein Inhibitor von Inosinmonophosphat-Dehydrogenase ist.

2. ω-Interferon zur Verwendung nach Anspruch 1, worin die Virenerkrankung eine Infektion mit HCV umfasst und das Individuum primäre oder sekundäre Resistenz gegen eine Behandlung mit einem Nicht-ω-Interferon zeigt.

3. ω-Interferon zur Verwendung nach Anspruch 1 oder Anspruch 2, worin die im Verfahren verabreichte therapeutisch wirksame Menge an ω-Interferon 135 bis 700 g pro Woche beträgt.

4. ω-Interferon zur Verwendung nach Anspruch 3, worin die Aktivität des ω-Interferons etwa 27 bis 420 Millionen internationale Einheiten (MIE) beträgt.

5. ω-Interferon zur Verwendung nach einem der Ansprüche 1 bis 4, worin der Inhibitor von Inosinmonophosphat-Dehydrogenase Ribavirin oder ein Ribavirin-Analog ist.

6. ω-Interferon zur Verwendung nach einem der Ansprüche 1 bis 4, worin der Inhibitor von Inosinmonophosphat-Dehydrogenase aus der aus Mycophenolsäure, Mycophenolat-Mofetil, Mycophenolsäure-Natrium, Aminothiadiazol, Thiophenfurin, Tiazofurin, Viramidin, VX-148, VX-497 und VX-944 bestehenden Gruppe ausgewählt ist.

7. ω-Interferon zur Verwendung nach einem der Ansprüche 5 und 6, worin der verabreichte Inhibitor von Inosinmonophosphat-Dehydrogenase einem menschlichen Individuum in einer therapeutisch wirksamen Dosis von 200 bis 4.800 mg pro Tag verabreicht wird.

8. ω-Interferon zur Verwendung nach einem der Ansprüche 1 bis 7, worin das verabreichte ω-Interferon parenteral, enterisch oder topisch verabreicht wird.

9. ω-Interferon zur Verwendung nach Anspruch 8, worin das ω-Interferon parenteral verabreicht wird.

10. ω-Interferon zur Verwendung nach Anspruch 9, worin das ω-Interferon intramuskulär, intraperitoneal, intravenös oder subkutan verabreicht wird.

11. ω-Interferon zur Verwendung nach Anspruch 10, worin das ω-Interferon 3-mal wöchentlich subkutan verabreicht wird.

12. ω-Interferon zur Verwendung nach Anspruch 9, worin das ω-Interferon mittels Injektion verabreicht wird.

13. ω-Interferon zur Verwendung nach Anspruch 12, worin das ω-Interferon durch eine oder mehrere tägliche Injektionen verabreicht wird.

14. ω-Interferon zur Verwendung nach einem der Ansprüche 1 bis 7, worin das ω-Interferon mittels Infusion verabreicht wird.

15. ω-Interferon zur Verwendung nach einem der Ansprüche 1 bis 7, worin das verabreichte ω-Interferon mit einer zeitlich kontrollierten Häufigkeit verabreicht wird.

16. ω-Interferon zur Verwendung nach Anspruch 15, worin das ω-Interferon unter Verwendung einer Vorrichtung verabreicht wird.

17. ω-Interferon zur Verwendung nach Anspruch 16, worin die Vorrichtung eine Pumpe umfasst.

18. ω-Interferon zur Verwendung nach Anspruch 17, worin die Vorrichtung ein Implantat im Individuum ist oder sich außerhalb des Individuums befindet.

19. ω-Interferon zur Verwendung nach Anspruch 18, worin die Vorrichtung ein Implantat in dem Individuum ist.

20. ω-Interferon zur Verwendung nach Anspruch 19, worin die Vorrichtung eine osmotische Pumpe umfasst.

21. ω-Interferon zur Verwendung nach einem der Ansprüche 1 bis 20, worin das ω-Interferon ein rekombinantes ω-Interferon ist.

22. ω-Interferon zur Verwendung nach einem der Ansprüche 1 bis 21, worin das Individuum ein Mensch ist.

23. Verwendung eines ω-Interferons und eines damit verbundenen therapeutischen Mittels bei der Herstellung eines Medikaments zur Behandlung einer Infektion mit einem Hepatitis-C-Virus (HCV) oder einem Gelbfiebervirus, worin das verbundene therapeutische Mittel ein Inhibitor von Inosinmonophosphat-Dehydrogenase ist.

## Revendications

1. Interféron oméga, pour utilisation dans le traitement de l'infection avec un Virus de l'Hépatite C (HCV) ou avec le virus de la fièvre jaune dans un sujet d'animal à sang chaud, où l'interféron oméga est un agent thérapeutique d'adjonction est administré au sujet, et où l'agent thérapeutique d'adjonction est un inhibiteur de l'inosine monophosphate déhydrogénase.

2. Interféron oméga pour utilisation selon la revendication 1, où la maladie virale comprend l'infection avec HCV, et le sujet présente une résistance primaire ou secondaire au traitement avec un interféron non-oméga.

3. Interféron oméga pour utilisation selon la revendication 1 ou la revendication 2, où la quantité thérapeutiquement efficace de l'interféron oméga administrée dans la méthode est de 135-700 microgrammes par semaine.

4. Interféron oméga pour utilisation selon la revendication 3, où l'activité de l'interféron oméga est d'environ 27-420 mUI.

5. Interféron oméga pour utilisation selon l'une quelconque des revendications 1 à 4, où l'inhibiteur de l'iosine monophosphate déhydrogénase est la ribavirine ou un analogue de ribavirine.

6. Interféron oméga pour utilisation selon l'une quelconque des revendications 1 à 4, où l'inhibiteur de l'inosine monophoaphate déhydrogénase est sélectionné dans le groupe consistant en acide mycophénolique, mycophénolate mofétile, sodium de l'acide mycophénolique, aminothiadiazole, thiophenfurine, tiazofurine, viramidine, VX-148, VX-497 et VX-944.

7. Interféron oméga selon l'une quelconque des revendications 5 ou 6, où l'inhibiteur de l'inosine monophosphate déhydrogénase est administré à un sujet humain à une dose thérapeutiquement efficace de 200 à 4800 milligrammes par jour.

8. Interféron oméga pour utilisation selon l'une quelconque des revendications 1 à 7, où l'interféron oméga administré est administré de manière parentérale, entérale ou topique.

9. Interféron oméga pour utilisation selon la revendication 8, où l'interféron oméga est administré de manière parentérale.

10. Interféron oméga pour utilisation selon la revendication 9, où l'interféron oméga est administré de manière intramusculaire, intrapéritonéale, intraveineuse ou sous-cutanée.

11. Interféron oméga pour utilisation selon la revendication 10, où l'interféron oméga est administré de manière sous-cutanée trois fois par semaine.

12. Interféron oméga pour utilisation selon la revendication 9, où l'interféron oméga est administré par injection.

13. Interféron oméga pour utilisation selon la revendication 12, où l'interféron oméga est administré par une ou plusieurs injections journalières.

14. Interféron oméga pour utilisation selon l'une quelconque des revendications 1 à 7, où l'interféron oméga est administré par infusion.

15. Interféron oméga pour utilisation selon l'une quelconque des revendications 1 à 7, où l'interféron oméga est administré selon un taux contrôlé sur le temps.

16. Interféron oméga pour utilisation selon la revendication 15, où l'interféron oméga est administré en utilisant un dispositif.

17. Interféron oméga pour utilisation selon la revendication 16, où le dispositif comprend une pompe.

18. Interféron oméga pour utilisation selon la revendication 17, où le dispositif est un implant dans ou est à l'extérieur du sujet.

19. Interféron oméga pour utilisation selon la revendication 18, où le dispositif est un implant dans le sujet.

20. Interféron oméga pour utilisation selon la revendication 19, où le dispositif comprend une pompe osmotique.

21. Interféron oméga pour utilisation selon l'une quelconque des revendications 1 à 20, où l'interféron oméga est un interféron oméga recombinant.

22. Interféron oméga pour utilisation selon l'une quelconque des revendications 1 à 21, où le sujet est un être humain.

23. Utilisation de l'interféron oméga et d'un agent thérapeutique d'adjonction dans la fabrication d'un médicament pour traiter l'infection par le Virus de l'Hépatite C (HCV) ou le virus de la fièvre jaune, où l'agent thérapeutique d'adjonction est un inhibiteur de l'inosine monophosphate déhydrogénase.
